# EUROPÄISCHE PATENTANMELDUNG

(11) **EP 3 760 698 A1**
(43) Veröffentlichungstag der Anmeldung: **06.01.2021**
(21) Anmeldenummer: 19183689.9
(22) Anmeldetag: 01.07.2019
(51) Int. Cl.: C11B 1/04, C11B 1/10, A23D 9/04, B01D 11/00, C12P 7/64

(54) **VERFAHREN ZUM EXTRAHIEREN VON LIPIDEN AUS EINER BIOMASSE**

(71) Anmelder: BDI Holding GmbH, 8074 Grambach (AT)
(72) Erfinder: Raudner, Robert, 8580 Köflach (AT); Kreinz, Marlène, 8077 Gössendorf (AT)
(74) Vertreter: Schwarz & Partner Patentanwälte OG

(57) **Zusammenfassung**

Ein Verfahren zum Extrahieren von Lipiden aus einer Biomasse umfasst die Schritte des Vermischens der Biomasse mit einem kurzkettigen, wasserlöslichen Alkohol und Bildens einer Suspension, des Hinzugebens eines wasserunlöslichen Extraktionsmittels zur Suspension und Bildens einer Extraktionsmittel/Alkohol-Biomasse-Mischung, des Abtrennens der Phase des wasserunlöslichen Extraktionsmittels von der Mischung und des Gewinnens der Lipide aus der Phase des wasserunlöslichen Extraktionsmittels. Durch dieses Verfahren wird insbesondere die Extraktion von Lipiden aus einer feuchter Biomasse mit einer guten Extraktionseffizienz ermöglicht.

## Beschreibung

Die Forschung, die zu den Ergebnissen führte, auf denen diese Anmeldung aufbaut, hat finanzielle Mittel vom siebenten EU-Forschungsrahmenprogramm (FP7/2007-2013) unter Fördervertrag Nr. 268208 erhalten.

Die Erfindung betrifft ein Verfahren zum Extrahieren von Lipiden aus einer Biomasse, insbesondere einer feuchten Biomasse.

Biomasse wird in den letzten Jahren in zunehmendem Maße als Ausgangsbasis für die Gewinnung von Wertstoffen herangezogen. Neben anderen Biomassen, beispielsweise Kaffeesud, stellt insbesondere Algenbiomasse eine wertvolle Quelle für chemische Rohstoffe, wie Fette und Öle, Proteine etc. dar, die im Hinblick auf die Schonung von Ressourcen für die Nahrungsmittelerzeugung und die Vermeidung von Kohlendioxidfreisetzung aus fossilen Brennstoffen gegenüber herkömmlichen Quellen als Rohstofflieferant zu bevorzugen ist.

Abhängig von den Wachstumsbedingungen können Algen einen hohen Protein-, Kohlenhydrat- oder Lipidgehalt aufweisen. So werden von den Algen bei Stickstoffmangel und gleichzeitigem Angebot an Kohlendioxid und Licht besonders Lipide als Speicherstoffe gebildet, wobei der Lipidgehalt bis zu 70% betragen kann.

Die in den Algen enthaltenen Lipide setzen sich in erster Linie aus Glycolipiden, Phospholipiden und Triglyceriden zusammen. Speicherlipide in Algen sind vor allem Triacylglyceride mit den Hauptfettsäuren C16:0, C16:1 und C18:1, welche vorwiegend zur Erzeugung von Kraftstoff, z.B. nach Umesterung als Biodiesel, genutzt werden können.

Lipide aus Biomasse wie Algen werden in erster Linie mittels Extraktion gewonnen. Hierbei kommt zum Beispiel das Verfahren von Bligh und Dyer, Folch oder Ryckebosch zur Anwendung, das auf einem aus Chloroform, Methanol und Wasser bestehenden System basiert.

Bei getrockneter Biomasse wird die Gewinnung von Fetten und Ölen üblicherweise durch Einsatz eines wasserunlöslichen Extraktionsmittels durchgeführt. Hexan hat sich hierbei als besonders geeignet erwiesen.

Ein besonderes Problem bei der Gewinnung von Lipiden ergibt sich jedoch vor allem bei der Verwendung feuchter Ausgangsmaterialien. Die hohe Viskosität von feuchter Biomasse führt bei entsprechendem Trockenrückstand zu einer schlechten Verarbeitbarkeit, insbesondere im Industriemaßstab, da sich das Ausgangsmaterial nicht bzw. nur ungenügend durch Pumpen transportieren lässt. So führt die klassische Extraktion bei feuchter, pastöser Biomasse nicht nur aufgrund der schlechten Verarbeitbarkeit zu Schwierigkeiten, sondern es kommt auch zu einer Beeinträchtigung der Extraktion, da sich anscheinend eine Hydrathülle um die einzelnen Zellen bildet, die dazu führt, das das Extraktionsmittel nicht in direkten Kontakt mit der Zelle, bzw. der Zellmembran und dem Inneren der Zelle kommt, wo sich die meisten Lipide befinden.

Das für die Extraktion von Lipiden aus trockener Biomasse häufig verwendete Hexan erweist sich für feuchte, d.h. wässrige, Biomasse als ungeeignet, da sich letztere in diesem Extraktionsmittel nicht löst bzw. mit diesem keine homogene Suspension erzielt wird. Die Verwendung wasserlöslicher Extraktionsmittel anstelle von Hexan führt zwar zu einer besseren Verarbeitbarkeit der feuchten Biomasse, doch resultiert das polare Lösungsmittel in erster Linie in einer Extraktion von polaren Lipiden, was in vielen Fällen nicht oder nicht ausschließlich gewünscht wird.

Um die bei Verwendung eines einzelnen Extraktions- oder Lösungsmittels auftretenden Probleme zu vermeiden, wurde versucht, Verfahren zu entwickeln, die eine Kombination eines polaren Lösungsmittels mit einem wasserunlöslichen Extraktionsmittel vorsehen.

Von M. Bokisch (Michael Bockisch, Handbuch der Lebensmitteltechnologie - Nahrungsfette und - öle, Verlag Eugen Umler, Stuttgart, 1993, ISBN 3-8001-5817-5) wird zur Extraktion von Rapsschrot ein Zweiphasensystem aus Hexan und einem Wasser/Ammoniak-Gemisch in Methanol vorgeschlagen, um den Glucosinolatgehalt des Rapsschrot-Mehls zu senken.

In der US 2013/0274490 A wird ein Verfahren zur Extraktion von Lipiden aus Algen beschrieben, bei dem trockene Algenpellets mehrmals in einem Methanol/Hexan-Gemisch bei 50°C eingeweicht werden und die Lösungsmittel zur Gewinnung von Algenöl jeweils verdampft werden. Nachteilig bei diesem Verfahren ist jedoch, dass die Lipide nicht durch einen einmaligen Kontakt mit Extraktionsmittel ausreichend aus der Biomasse gelöst werden, sondern dass es mehrerer Durchgänge bedarf, um eine sinnvolle Extraktionseffizienz zu erzielen, was größere Lösungsmittelmengen und damit verbunden auch mehr Energie für die Gewinnung der Lipide aus dem Lösungsmittel erforderlich macht.

Die Erfindung versucht, die oben genannten Probleme zu überwinden, und stellt sich die Aufgabe, ein Verfahren zum Extrahieren von Lipiden aus einer Biomasse bereitzustellen, das insbesondere bei einer feuchten Biomasse eine gute Verarbeitbarkeit gewährleistet sowie die durch den Wassergehalt beeinträchtigte Extrahierbarkeit durch wasserunlösliche Extraktionsmittel beseitigt. Außerdem soll das Verfahren in einem einzigen Durchgang eine wirtschaftlich sinnvollen Extraktionseffizienz erreichen und zudem ermöglichen, die Extraktion von polaren Lipiden zu vermeiden, die für bestimmte Anwendungen unerwünscht sind.

Diese Aufgabe wird erfindungsgemäß bei einem Verfahren der eingangs genannten Art dadurch gelöst, dass es die folgenden Schritte umfasst:
- Vermischen der Biomasse mit einem kurzkettigen, wasserlöslichen Alkohol, vorzugsweise Methanol oder Ethanol, und Bilden einer Suspension,
- Hinzugeben eines wasserunlöslichen Extraktionsmittels, vorzugsweise n-Hexan oder iso-Hexan, zur Suspension und Bilden einer Extraktionsmittel/Alkohol-Biomasse-Mischung,
- Abtrennen der Phase des wasserunlöslichen Extraktionsmittels von der Mischung,
- Gewinnen der Lipide aus der Phase des wasserunlöslichen Extraktionsmittels.

Handelt es sich beim Ausgangsmaterial für die Extraktion um eine feuchte Biomasse, z.B. Algenbiomasse, so besitzt diese im Normalfall aufgrund ihres entsprechend geringen Trockenrückstands eine derart hohe Viskosität, dass sie in herkömmlichen Extraktionsvorrichtungen nicht oder nur schwer und mit hohem apparativem Aufwand zu verarbeiten ist. Aus diesem Grund wird in den meisten Fällen trockene Biomasse, also Biomasse mit einem sehr hohen Trockenrückstand eingesetzt, was jedoch einen meist energie- und kostenintensiven Trocknungsvorgang erforderlich macht.

Durch das erfindungsgemäße Vermischen der Biomasse mit einem kurzkettigen, wasserlöslichen Alkohol und Bilden einer Suspension im ersten Verfahrensschritt wird jedoch nicht nur aus getrockneter Biomasse sondern vorteilhafterweise auch aus einer pastösen, wasserhältigen Biomasse hoher Viskosität ein leicht verarbeitbares, handhabbares Ausgangsmaterial, das sich ohne große Probleme und mit geringem Aufwand in konventionellen Vorrichtungen transportieren und pumpen lässt. Außerdem wird durch die homogene Verteilung der Biomasse in der Suspension eine gute Extraktionseffizienz begünstigt, sodass ein einziger Extraktionsschritt, d.h. ein einmaliges Hinzufügen von Lösungsmittel zur Biomasse, ausreicht.

Durch diese erfindungsgemäße Maßnahme wird nicht nur die Verarbeitbarkeit feuchter Biomasse gewährleistet, sondern es können auch die Kosten und die Energie für die sonst erforderliche Biomasse-Trocknung sowie für mehrere Extraktionsdurchgänge eingespart werden.

In einer bevorzugten Ausfuhrungsform wird die Biomasse mit mindestens 50 Gew.-% Alkohol, bezogen auf die Biomasse, vermischt.

Bevorzugt wird die Biomasse mit einer derartigen Menge an Alkohol vermischt, dass der Trockenrückstand der Biomasse zwischen 2 und 25% beträgt. Der Trockenrückstand entspricht dem Trockenrückstand TS, wie er gemäß EN 12880 bestimmt wird.

Zweckmäßigerweise wird die Bildung der Suspension, also bis sich eine homogende Verteilung eingestellt hat, durch Rühren der aufgeschlämmten Biomasse erleichtert.

Als kurzkettiger, wasserlöslicher Alkohol wird vorteilhafterweise ein Alkohol aus der Gruppe, bestehend aus Methanol, Ethanol, Propanol und iso-Propanol, verwendet, vorzugsweise Methanol oder Ethanol.

Die gebildete alkoholische, z.B. methanolische, Biomasse wir erfindungsgemäß in einem nächsten Schritt mit einem wasserunlöslichen Extraktionsmittel vermischt. Als wasserunlösliches Extraktionsmittel wird vorteilhafterweise ein Extraktionsmittel aus der Gruppe, bestehend aus n-Hexan, iso-Hexan, Heptan und Petrolether, vorzugsweise n-Hexan oder iso-Hexan, verwendet.

In einer bevorzugten Ausführungsform wird das wasserunlösliche Extraktionsmittel im Gewichtsverhältnis von 1:2 bis 2:1, vorzugsweise 1:1, bezogen auf die Biomasse, zur Suspension hinzugefügt.

Da es aufgrund der alkoholischen Suspension zu einem besseren Kontakt der nicht mischbaren Phasen (Wasser / wasserunlösliches Extraktionsmittel) kommt, findet nun nicht nur bei trockener sondern auch bei feuchter Biomasse eine verbesserte Extraktion von unpolaren Lipiden statt.

Abhängig von der Zusammensetzung der Biomasse kann sich aufgrund von dessen phasenvermittelnder Wirkung eine derartige Suspension bilden, dass das Gemisch quasi einphasig wird. Hierdurch kommt es zu einem direkten Kontakt der Biomasse mit dem Extraktionsmittel, wodurch sich die Extraktionseffizienz noch weiter erhöht. Bildet sich aufgrund der Natur der verwendeten Biomasse keine solche Suspension, kann durch Erwärmen des Gemisches ein einphasiges System hergestellt werden. Bei einer Temperaturerhöhung stellt sich nämlich bei der Verwendung eines binären Alkohol/wasserunlösliches Extraktionsmittel-Gemisches, z.B. Methanol/Hexan, ein einphasiges System ein.

Gemäß einer bevorzugten Ausführungsform wird deshalb die gebildete Extraktionsmittel/Alkohol-Biomasse-Mischung erhitzt, vorzugsweise auf Siedetemperatur.

Vorzugsweise wird die Extraktionsmittel/Alkohol-Biomasse-Mischung vor dem Abtrennungsschritt abgekühlt, damit es (wieder) zu einer Phasentrennung kommt.

Da das erfindungsgemäße Verfahren, wie oben ersichtlich, in Bezug auf feuchte Biomasse besondere Vorteile aufweist, wird das Verfahren gemäß einer bevorzugten Ausführungsform an einer Biomasse angewandt, die einen Trockenrückstand zwischen 5 und 95 Gew.-%, vorzugsweise zwischen 10 und 60 Gew.-%, aufweist.

Aufgrund der sich bildenden Suspension ist eine Trennung in eine wasserunlösliche Phase, in der die (unpolaren) Lipide gelöst sind, in eine Wasser/Methanol-Phase sowie eine Feststoffphase (unlöslicher Biomasseanteil) mittels eines Dreiphasendekanters besonders geeignet.

Daher wird in einer bevorzugten Ausführungsform die Extraktionsmittelphase mittels Dreiphasendekanter von der Alkohol/Wasser-Phase und der Feststoffphase der Mischung abgetrennt.

Um die Lipide zu erhalten, wird das wasserunlösliche Extraktionsmittel typischerweise verdampft, wobei es nach der Kondensation wieder im Verfahren eingesetzt werden kann. Der Alkohol wird zweckmäßigerweise aus der Alkohol/Wasser-Phase, z.B. mittels Destillation, zurückgewonnen und ebenfalls wieder im Verfahren verwendet.

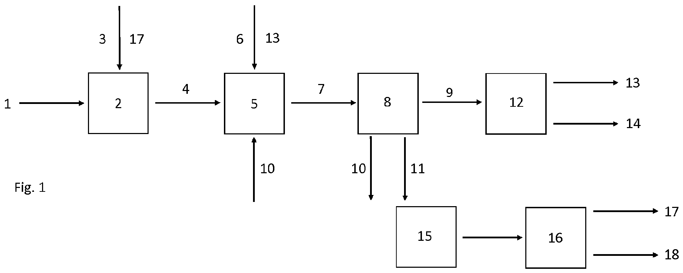

Die Erfindung wird nachfolgend anhand von Beispielen und der Zeichnung näher erläutert, wobei Fig. 1 ein Blockdiagramm einer Ausführungsform des erfindungsgemäßen Verfahrens zeigt.

Wie in Fig. 1 gezeigt, wird die Biomasse 1, z.B. über eine Förderschnecke, in einen Rührreaktor 2 überführt, dort mit einem kurzkettigen, wasserlöslichen Alkohol, vorzugsweise Methanol oder Ethanol, versetzt und bis zur vollständigen Durchmischung gerührt. Anschließend wird die Biomasse-Suspension 4 in einen beheizbaren, mit Rührer ausgestatteten Reaktor 5 gepumpt, in den ein wasserunlösliches Extraktionsmittel, vorzugsweise n-Hexan oder iso-Hexan, eingebracht wird.

Die gebildete Extraktionsmittel/Alkohol-Biomasse-Mischung wird im Reaktor 5, je nach Bedarf bei Raumtemperatur oder bei Siedetemperatur (unter Rückfluss), für die Dauer der Reaktionszeit (Extraktionszeit) gerührt. Danach wird das Reaktionsgemisch 7 (die extrahierte Extraktionsmittel/Alkohol-Biomasse-Mischung) zu einem Dreiphasendekanter 8 (Trikanter) befördert und dort in eine lipidangereicherte Extraktionsmittelphase 9, eine Biomasse-Feststoffphase 10 und eine Alkohol/Wasser-Phase 11 aufgetrennt.

Die Extraktionsmittelphase 9 wird einer Destillation 12 unterworfen, um das Extraktionsmittel 13 vom Extrakt 14 zu trennen. Das Extraktionsmittel 13 kann erneut zur Extraktion im Reaktor 5 eingesetzt werden. Der Extrakt 14 wird der beabsichtigten Verwendung, z.B. einem Veresterungs- oder Umesterungsprozess, zugeführt.

Wahlweise kann die Biomasse 10 teilweise zum Reaktor 5 rückgeführt werden, um darin verbliebenes lipidhaltiges Extraktionsmittel auszuwaschen und das Extraktionsergebnis damit zusätzlich zu steigern. Die Alkohol/Wasser-Phase 11 wird mittels eines Filters 15 von gegebenenfalls noch vorhandenem Feststoff befreit und einer Destillation 16 zur Abtrennung des Alkohols 17 von der wässrigen Phase 18 zugeführt. Die wässrige Phase 18, die Kohlenhydrate, Proteine und polare Lipide enthält, kann z.B. einer Biogas-Anlage zugeführt werden.

### Beispiele:

Bei den in den Beispielen eingesetzten Biomassen wurden jeweils der Trockenrückstand (TS) gemäß EN 12880 und der Glühverlust (organischer Trockenrückstand oTS) gemäß EN 12879 bestimmt.

Als Referenzmethode für die Bestimmung des Lipidgehalts wird die Algenbiomasse gefriergetrocknet und mittels einer Wirbelschichtextraktion (Ikafex) mit n-Hexan extrahiert, um die unpolaren Lipide (z.B. Triglyceride) zu erhalten.

Bezüglich der Extraktion von Lipiden aus Kaffesud wurde für die Referenzanalytik mittels Wirbelschichtextraktion ein Teil des feuchten Kaffeesuds entnommen und im Trockenschrank für 3 Stunden bei 105°C getrocknet.

Die Extraktionen wurden mit n-Hexan durchgeführt. Dazu wurden je 6 g Probe eingewogen und mit je 150 ml n-Hexan in 5 Zyklen extrahiert. Mit Hilfe eines Rotationsverdampfers wurde das Hexan der mit Extrakt beladenen Hexanphasen abdestilliert und der Extrakt im Anschluss bis zur Gewichtskonstanz getrocknet.

### 1. Extraktion feuchter Algen-Biomasse mit n-Hexan unter Rückfluss im Reaktor (Vergleichsbeispiel)

Die Biomasse und das n-Hexan wurden im Verhältnis 1:1 in einen 2 Liter-Doppelmantel-Glasreaktor überführt, mittels eines Schrägblattrührers mit Rührwerk (Heidolph RFZ 22102 control) gerührt (346 U/min) und mittels eines Thermostats (Lauda Ecoline Star Edition RE 307) auf Siedetemperatur von 72°C erhitzt. Nach einer Reaktionszeit von 2 Stunden wurde die Temperatur des Reaktionsgemisches auf unter 30 °C erniedrigt und die Hexanphase in eine 2 Liter-Schottflasche überführt. Die Biomasse wurde 4 x mit je 50 ml n-Hexan gewaschen und dieses Hexan mit der ersten Hexanphase vereinigt. Die Biomasse wurde mit 1 Liter Wasser aufgeschlämmt und so aus dem Reaktor abgelassen. Mit Hilfe eines Scheidetrichters wurden die letzten Reste an Hexan abgetrennt und ebenfalls mit der ersten Hexanphase vereinigt.

Die gesamte Hexanphase wurde erst durch einen 2,5 µm Filter und anschließend durch einen 0,7 µm Filter mit Hilfe einer Vakuumpumpe (Vacubrand CVC 2) filtriert. Anschließend wurde das n-Hexan mit Hilfe eines Rotationsverdampfers (Heidolph Laborota 400 efficient WB eco) bei 55 °C und 400 mbar vom Algenextrakt abgetrennt. Der Extrakt wurde anschließend im Trockenschrank (Binder) bei 75 °C bis zur Gewichtskonstanz getrocknet.

| **Ergebnisse Beispiel 1** | |
|---|---|
| TS [%] | 15,6 |
| organische TS [%] | 67,9 |
| w% _{Laborreferenzwert Ölextrakt bez. auf organische TS} [%] | 2,59 |
| m _{BM} [g] | 405,2 |
| m _{n-Hexan} [g] | 408,1 |
| m_{Algen TS} [g] | 63,2 |
| m_{Algen oTS} [g] | 42,9 |
| m _{Extrakt} [g] | 0,5399 |
| **w % _{Extrakt bez. auf oTS} [%]** | **1,26** |
| Ausbeute im Vergleich zu Referenzwert Labor [%] | 48,6 |

| | |
|---|---|
| TS ......Trockenrückstand oTS ......... organischer Trockenrückstand (Glühverlust) m ............Gewicht BM .......Biomasse | |

Da sich die Algen-Biomasse und das n-Hexan nicht mischten, kam es nur an der Phasengrenze der beiden Schichten zu einem Kontakt des Extraktionsmittels mit dem zu extrahierenden Gut.
Die Ausbeute ist ca. halb so groß wie mit der Referenzmethode ermittelt.

### 2. Wiederholung Versuch 1 mit längerer Extraktionszeit (Vergleichsbeispiel)

Dieser Versuch wurde mit Ausnahme einer längeren Reaktionszeit von 4 Stunden analog zu dem Versuch in Beispiel 1 durchgeführt.

| **Ergebnisse Beispiel 2** | |
|---|---|
| TS [%] | 15,9 |
| organische TS [%] | 67,9 |
| w% _{Laborreferenzwert Ölextrakt bez. auf organische TS} [%] | 2,59 |
| m _{BM} [g] | 414,4 |
| m _{n-Hexan} [g] | 414,9 |
| m_{Algen TS} [g] | 65,9 |
| m_{Algen oTS} [g] | 44,7 |
| m _{Extrakt} [g] | 0,5630 |
| **w% _{Extrakt bez. auf oTS} [%]** | **1,26** |
| Ausbeute im Vergleich zu Referenzwert Labor [%] | 48,6 |

Bei diesem Versuch ergab sich eine mit Beispiel 1 vergleichbare Extraktausbeute. Somit zeigt sich, dass auch bei längerer Extraktionszeit eine Erhöhung der Extraktausbeute mit n-Hexan allein nicht möglich ist.

### 3. Extraktion von feuchter Biomasse mit einer Mischung aus Methanol und n-Hexan

Um die Probleme, die bei der Extraktion mit n-Hexan entstehen, zu lösen, wurde eine Extraktion mit einem zweiten Extraktionsmittel entwickelt. Methanol wurde als weiteres Extraktionsmittel eingesetzt, weil es sehr gut wasserlöslich ist und somit in der Lage ist, die Biomasse gut aufzuschlämmen, um eine Suspension zu bilden. Man konnte sich des weiteres die Tatsache zu Nutze machen, dass n-Hexan und Methanol bei Raumtemperatur nicht oder nur sehr schlecht mischbar sind, sich aber ab einer Temperatur von 31 °C ein einphasiges System einstellt. Außerdem stellt sich bei der Mischung eine geringere Siedetemperatur (56 °C) ein, als die jeweiligen Reinsubstanzen aufweisen (n-Hexan T_{Siede}: 69 °C, Methanol T_{Siede}: 65 °C), was auch zu einer Energieeinsparung führt.

Basierend auf der verwendeten Referenzanalytik und der US-Patentanmeldung 2013/0274490 A wurde in einem ersten Versuch feuchte Algenbiomasse vorgelegt, ein Gemisch aus Methanol und n-Hexan im Gewichtsverhältnis 1:1 hinzugefügt und im Wirbelschichtverfahren extrahiert. In einem zweiten Versuch wurde die Biomasse vor der Extraktion mit der gleichen Menge Methanol aufgeschlämmt und anschließend mit der gleichen Menge n-Hexan vermischt und extrahiert. Überraschenderweise wurde festgestellt, dass der zweite Versuch, bei welchem die Biomasse im Vorfeld mit Methanol aufgeschlämmt wurde, eine signifikant höhere Ausbeute an Extrakt lieferte.

| **Ergebnisse Beispiel 3** | | |
|---|---|---|
| | Versuch 1 | Versuch 2 |
| Einwaage BM [g] | 20,4 | 20,8 |
| Einwaage MeOH [g] | 59,3 | 59,3 |
| Einwaage n-Hexan [g] | 49,5 | 49,5 |
| Extrakt bez. auf oTS [g] | 0,9 | 1,45 |

### 4. Extraktion feuchter Algen-Biomasse mit Methanol und n-Hexan unter Rückfluss im Reaktor

Bei diesem Versuch wurde die Algenbiomasse in den Doppelmantel überführt und mit so viel Methanol versetzt, dass sich eine homogene, gut rührbare Suspension einstellte, und 1 Stunde bei Raumtemperatur gerührt. Anschließend wurde n-Hexan im Verhältnis 1:1 zur Biomasse hinzugefügt und auf Siedetemperatur von 56 °C erhitzt. Nach einer Reaktionszeit von 2 Stunden wurde das Reaktionsgemisches auf unter 30 °C abgekühlt, über das Bodenablassventil des Reaktors abgelassen und mittels einer Zentrifuge (Hitachi CR22N) mit 3820 g bei 25 °C für 6 min separiert.

Die Hexanphase und der Extrakt wurden wie in Beispiel 1 beschrieben aufgearbeitet.

| **Ergebnisse Beispiel 4** | |
|---|---|
| TS [%] | 15,9 |
| organische TS [%] | 67,9 |
| w% _{Laborreferenzwert Ölextrakt bez. auf organische TS} [%] | 2,59 |
| m _{BM} [g] | 403,7 |
| m _{n-Hexan} [g] | 401,4 |
| m _{Methanol} [g] | 239,3 |
| m_{Algen TS} [g] | 64,2 |
| m_{Algen oTS} [g] | 43,6 |
| m _{Extrakt} [g] | 2,3258 |
| **w % _{Extrakt bez. auf oTS} [%]** | **5,33** |
| Ausbeute im Vergleich zu Referenzwert Labor [%] | 205,8 |

Die Biomasse vermischte sich sehr gut mit dem Methanol. Die Durchmischung der Biomasse und der Kontakt mit dem Extraktionsmittel (Hexan) war dadurch viel besser gegeben. Des Weiteren konnte das ganze Extraktionsgemisch aus dem Reaktor abgelassen und anschließend mittels Zentrifugation separiert werden. Die Extraktausbeute war ca. 4 x höher als bei Beispiel 1 und zeigte eine doppelt so hohe Ausbeute wie die Referenzmethode.

### 5. Extraktion feuchter Algen-Biomasse mit Methanol und n-Hexan bei Raumtemperatur im Reaktor

Die Biomasse wurde in den Reaktor eingewogen, mit MeOH versetzt und 1 Stunde bei RT (25°C) mittels einem Schrägblattrührer bei 400 U/min gerührt. Es bildet sich schnell eine homogene Suspension. Anschließend wurde die entsprechende Menge n-Hexan hinzugefügt und bei erhöhter Drehzahl des Rührers von 550 U/min für 2 Stunden bei RT (25°C) weitergerührt.

Mithilfe von MeOH als kurzkettiger, wasserlöslicher Alkohol und Lösungsvermittler bildete sich rasch eine homogene Suspension. Auch das n-Hexan mischte sich schnell ein und es bildete sich trotz Reaktion bei RT insgesamt ein homogenes Reaktionsgemisch. Auffallend war, dass sich nach der Standzeit von 4 Tagen keinerlei leichte Phase gebildet hatte.

Das Reaktionsgemisch wurde mithilfe einer Zentrifuge, bei 6000 U/min für 3 min, aufgetrennt. Die Hexanphase wurde mit VE (vollentsalztes)-Wasser gewaschen und anschließend mithilfe eines 0,7µm Filters filtriert.

Nachfolgend wurde das Hexan wie in den vorhergehenden Beispielen abdestilliert und der Extrakt im Trockenschrank bei 70°C bis zur Gewichtskonstanz getrocknet.

| **Ergebnisse Beispiel 5** | |
|---|---|
| TS [%] | 21,6 |
| organische TS [%] | 74,3 |
| w% _{Laborreferenzwert Ölextrakt bez. auf organische TS} [%] | 3,9 |
| m _{BM} [g] | 425,9 |
| m _{n-Hexan} [g] | 425,6 |
| m _{Methanol} [g] | 605,5 |
| m_{Algen TS} [g] | 92,0 |
| m_{Algen oTS} [g] | 68,4 |
| m _{Extrakt} [g] | 4,4138 |
| **w % _{Extrakt bez. auf oTS} [%]** | **6,45** |
| Ausbeute im Vergleich zu Referenzwert Labor [%] | 165,5 |

### 6. Extraktion feuchter Algen-Biomasse mit Methanol und n-Hexan bei Siedetemperatur im Technikumsmaßstab

Die BM wurde mit der entsprechenden Menge MeOH vermischt, in den Reaktor gepumpt und mittels eines Propellerrührers bei 655 U/min für 1 Stunde gerührt. Anschließend wurde die entsprechende Menge n-Hexan hinzugefügt und für ca. 1,5 Stunden bei Siedetemperatur des Reaktionsgemisches gerührt.

Nach Ende der Reaktionszeit wurde das Reaktionsgemisch mit Hilfe eines 3-PhasenSeparators (einem gasdichten Trikanter, Typ Z23 von Flottweg) aufgetrennt. Die Biomasse wurde anschließend mit frischem n-Hexan aufgeschlämmt und im 2. Durchlauf noch einmal separiert, um die Extraktionsausbeute zusätzlich zu erhöhen.

Das Hexan der mit Algenextrakt beladene Hexan-Phase wurde direkt nach der Separation mittels eines 50 Liter - Rotationsverdampfers abdestilliert.
Bei diesem Versuch entfiel das Trocknen der Extraktphase.

| **Ergebnisse Beispiel 6** | |
|---|---|
| TS [%] | 22,0 |
| organische TS [%] | 67,5 |
| w% _{Laborreferenzwert Ölextrakt bez. auf organische TS} [%] | 4,2 |
| m _{BM} [kg] | 242,1 |
| m _{n-Hexan} [kg] | 243,0 |
| m _{Methanol} [kg] | 319,8 |
| m_{Algen TS} [kg] | 53,3 |
| m_{Algen oTS} [kg] | 36,0 |
| m _{Extrakt} [kg] | 9,5 |
| **w % _{Extrakt bez. auf oTS} [%]** | **26,4** |
| Ausbeute im Vergleich zu Referenzwert Labor [%] | 628,3 |

Wie die vorstehenden Beispiele zeigen, wird beim erfindungsgemäßen Verfahren bei einer feuchten Biomasse eine gute Verarbeitbarkeit erzielt und auch schon bei einem einzigen Durchgang eine höhere Extraktionseffizienz erreicht als bei Extraktion mit einem wasserunlöslichen Extraktionsmittel allein oder mit einem fertigen Gemisch aus einem wasserunlöslichen Extraktionsmittel und einem kurzkettigen Alkohol.

### 7. Extraktion von feuchtem Kaffeesud mit Methanol und n-Hexan unter Rückfluss im Reaktor

Die Biomasse wurde im 2 Liter-Doppelmantelreaktor vorgelegt, mit der entsprechenden Menge Methanol vermischt und bei RT (25°C) für 1 Stunde bei 400U/min gerührt. Anschließend wurde n-Hexan hinzugefügt und das Reaktionsgemisch auf Siedetemperatur (ca. 52°C - 56°C) erhitzt. Die Extraktion erfolgte unter Rückfluss. Nach Ablauf der Reaktionszeit von 2 Stunden wurde das Reaktionsgemisch auf <40°C abgekühlt, über einen 50µm Beutelfilter und anschließend über einen 0,7µm Filter filtriert. Die mit Extrakt beladenen Hexanphase wurden mit Hilfe des Rotationsverdampfers (bei 60°C und 400 mbar, Enddruck 100 mbar) abgedampft und der Extrakt im Anschluss im Trockenschrank bei 80°C bis zur Gewichtskonstanz getrocknet.

| **Ergebnisse Beispiel 7** | |
|---|---|
| TS [%] | 44,87 |
| organische TS [%] | 99,06 |
| w% _{Laborreferenzwert Ölextrakt bez. auf organische TS} [%] | 17,86 |
| m _{BM} [g] | 401,6 |
| m _{n-Hexan} [g] | 401,0 |
| m _{Methanol} [g] | 567,9 |
| m_{Algen TS} [g] | 180,2 |
| m_{Algen oTS} [g] | 178,5 |
| m _{Extrakt} [g] | 29,9 |
| **w% _{Extrakt bez. auf oTS} [%]** | **16,8** |
| Ausbeute im Vergleich zu Referenzwert Labor [%] | 93,8 |

## Patentansprüche

1. Verfahren zum Extrahieren von Lipiden aus einer Biomasse, insbesondere einer feuchten Biomasse, umfassend die folgenden Schritte:
- Vermischen der Biomasse mit einem kurzkettigen, wasserlöslichen Alkohol, vorzugsweise Methanol oder Ethanol, und Bilden einer Suspension,
- Hinzugeben eines wasserunlöslichen Extraktionsmittels, vorzugsweise n-Hexan oder iso-Hexan, zur Suspension und Bilden einer Extraktionsmittel/Alkohol-Biomasse-Mischung,
- Abtrennen der Phase des wasserunlöslichen Extraktionsmittels von der Mischung,
- Gewinnen der Lipide aus der Phase des wasserunlöslichen Extraktionsmittels.

2. Verfahren nach Anspruch 1, **dadurch gekennzeichnet, dass** die Biomasse mit mindestens 50 Gew.-% Alkohol, bezogen auf die Biomasse, vermischt wird.

3. Verfahren nach Anspruch 1 oder 2, **dadurch gekennzeichnet, dass** die Biomasse mit einer derartigen Menge an Alkohol vermischt wird, dass der Trockenrückstand der Biomasse zwischen 2 und 25% beträgt.

4. Verfahren nach einem der Ansprüche 1 bis 3, **dadurch gekennzeichnet, dass** das wasserunlösliche Extraktionsmittel im Gewichtsverhältnis von 1:2 bis 2:1, vorzugsweise 1:1, bezogen auf die Biomasse, zur Suspension hinzugefügt wird.

5. Verfahren nach einem der Ansprüche 1 bis 4, **dadurch gekennzeichnet, dass** die gebildete Extraktionsmittel/Alkohol-Biomasse-Mischung erhitzt wird, vorzugsweise auf Siedetemperatur.

6. Verfahren nach Anspruch 5, **dadurch gekennzeichnet, dass** die Extraktionsmittel/Alkohol-Biomasse-Mischung vor dem Abtrennungsschritt abgekühlt wird.

7. Verfahren nach einem der Ansprüche 1 bis 6, **dadurch gekennzeichnet, dass** die Biomasse einen Trockenrückstand zwischen 5 und 95 Gew.-%, vorzugsweise zwischen 10 und 60 Gew.-%, aufweist.

8. Verfahren nach einem der Ansprüche 1 bis 7, **dadurch gekennzeichnet, dass** die Extraktionsmittelphase mittels Dreiphasendekanter von der Alkohol/Wasser-Phase und der Feststoffphase der Mischung abgetrennt wird.
